Europäisches Patentamt

European Patent Office

Office européen des brevets

(19)

(11) Veröffentlichungsnummer: **0 258 535**
**A2**

# (12) EUROPÄISCHE PATENTANMELDUNG

(21) Anmeldenummer: **87107244.3**

(22) Anmeldetag: **19.05.87**

(51) Int. Cl.⁴: **C07C 51/145** , C07C 55/21 , B01J 31/24

(30) Priorität: **23.08.86 DE 3628664**

(43) Veröffentlichungstag der Anmeldung:
**09.03.88 Patentblatt 88/10**

(84) Benannte Vertragsstaaten:
**AT BE CH DE ES FR GB IT LI NL SE**

(71) Anmelder: **Degussa Aktiengesellschaft**
**Weissfrauenstrasse 9**
**D-6000 Frankfurt am Main 1(DE)**

(72) Erfinder: **Andrade, Juan, Dr.**
**545 Barnett Place**
**Ridgewood N.J. 07450(US)**
Erfinder: **Köhler, Klaus**
**Kettelerstrasse 37**
**D-6452 Hainburg(DE)**
Erfinder: **Prescher, Günter, Dr.**
**Liesingstrasse 2**
**D-6450 Hanau 9(DE)**

(54) **Verfahren zur Herstellung von 1,12-Dodecandisäure II.**

(57) Die Erfindung betrifft ein Verfahren zur Herstellung von 1,12-Dodecandisäure durch Hydroformylierung einer ungesättigten Carboxylverbindung in Gegenwart eines Rhodiumkatalysators und Oxidation des dabei gebildeten Aldehyds mit Sauerstoff bei dem man Undecyl-10-en-säure in Gegenwart von Hydridotristriphenylphosphin-rhodium-carbonyl kombiniert mit Triphenylphosphin und/oder Triphenylphosphit hydroformyliert, und die dabei gebildete 11-Formylundecansäure in gegenwart von anorganischen oder organischen Co(II)-salzen mit Sauerstoff oxidiert.

EP 0 258 535 A2

## Verfahren zur Herstellung von 1,12-Dodecandisäure II.

Die Erfindung betrifft ein Verfahren zur Herstellung von 1,12-Dodecandisäure.

Es ist bekannt, diese Säure durch Oxidation und Verseifung des bei der Hydroformylierung von Methylundecylenat entstehenden Methyl-11-aldehydo-undecanoats zu gewinnen (H. Adkins, G. Krsek, JACS, Vol. 70, Januar 1948, S. 383 - 386).

Ebenfalls nur an einer Doppelbindung hydroformylierte Produkte erhält man, wenn man 1,9 Decadien in Gegenwart einer Rhodiumverbindung als Katalysator mit Kohlenmonoxid und Wasserstoff umsetzt.
Es entstehen Undecenale (DE-OS 2724484), die nach Oxidation und Veresterung als Ausgangsverbindung gemäß den Verfahren von Adkins und Krsek eingesetzt werden können.

Es wurde nun ein Verfahren zur Herstellung 1,12-Dodecandisäure durch Hydroformylierung einer ungesättigten Carboxylverbindung in Gegenwart eines Rhodiumkatalysators und Oxidation des dabei gebildeten Aldehyds mit Sauerstoff gefunden, das dadurch gekennzeichnet ist, daß man Undecyl-10-ensäure in Gegenwart von Hydridotristriphenylphosphin-rhodium-carbonyl kombiniert mit Triphenylphosphin und/oder Triphenylphosphit hydroformyliert und die dabei gebildete 11-Formylundecansäure in Gegenwart von anorganischen oder organischen Co-(II)-salzen mit Sauerstoff oxidiert.

Die 11-Formylundecansäure wird mit einem Gasgemisch aus Wasserstoff und Kohlenmonoxid in Gegenwart eines Katalysators hydroformyliert. Die Umsetzung erfolgt zweckmäßigerweise bei Temperaturen von 80 bis 140 °C, vorzugsweise bei Temperaturen von 80 bis 120 °C. Der Druck kann weitgehend beliebig gewählt werden, jedoch ist es zweckmäßig, im Druckbereich von 1 bis 12 bar zu arbeiten. Bevorzugt werden Drücke von 2 bis 8 bar.

Zweckmäßig ist es, die zur Umsetzung mindestens notwendigen stöchiometrischen Mengen, vorzugsweise jeweils die 1,05 bis 2,5-fache Mengen, Wasserstoff und Kohlenmonoxid zu verwenden, wobei das Molverhältnis Wasserstoff zu Kohlenmonoxid weitgehend beliebig gewählt werden kann, vorzugsweise jdoch zwischen 0,5 zu 1,0 bis 1,0 zu 0,5 liegt.

Bei der Hydroformylierung dient als Katalysator Hydridotris-triphenylphosphin-rhodium-carbonyl kombiniert mit Triphenylphosphin und beziehungsweise oder Triphenylphosphit. Derartige Katalysatoren sind in der DE-AS 1793069 beschrieben. Vorzugsweise werden bei der Druchführung des erfindungsgemäßen Verfahrens je Gewichtsteil Undecyl-10-en-säure 0,002 bis 0,01 Gewichtsteile des Hydridotris-triphenylphosphin-rhodium-carbonyls und insgesamt 0,02 bis 0,3 Gewichtsteile des Triphenylphosphins und/oder Triphenylphosphits angewendet.

Die bei der Hydroformylierung entstehende 11-Formylundecansäure wird in einer bevorzugten Variante des erfindungsgemäßen Verfahrens aus dem Reaktionsgemisch abgetrennt und anschließend bei 10 bis 100 °C, bevorzugt 40 bis 90 °C, in Gegenwart eines organischen oder anorganischen Co(II)-salzes mit einem sauerstoffhaltigen Gas oder reinem Sauerstoff bis zur Beendigung der Umsetzung oxidert.
Bevorzugt werden je Gewichtsteil 11-Formylundecansäure 0,005 bis 0,09 Gewichtsteile des Co(II)-salzes, vorzugsweise 0,01 Gewichtsteile, insbesondere Co(II)-acetat, verwendet.

In einer besonderen Ausführungsform führt man die Oxidation in Gegenwart einer Percarbonsäure in einer Menge von 0,002 bis 0,2 Gewichtsteile, bezogen auf die zu oxidierende Verbindung, zu.

Bevorzugt eingesetzt werden Peressigsäure und Perpropionsäure.

Die entstehende 1,12-Dodecandisäure wird nach Beendigung der Umsetzung und Abkühlen aus dem Oxidationsgemisch mit bekannten Maßnahmen abgetrennt.

Die Oxidation wird in einer besonders geeigneten Variante direkt in der bei der Hydroformylierung anfallenden Lösung durchgeführt, wobei die sonstigen Maßnahmen, wie oben beschrieben, bestehen bleiben.

Eine bevorzugte Ausführungsform beinhaltet die Verwendung eines inerten organischen Lösungsmittels, bevorzugt eines aromatischen wie z. B. Benzol oder Toluol, in dem bei der Hydroformylierung bzw. Oxidation vorgelegten Gemisch.

Beispiele

Beispiel 1

Undecyl-10-ensäure (64,5 g) wurde in 150 ml Toluol in einem Rührautoklaven mit 8,4 g Triphenylphosphin und 0,44 g Hydridotris-triphenylphosphin-rhodium-carbonyl vorgelegt. Dann wurde unter 6 bar Druck eine Mischung aus gleichen Volumenteilen Wasserstoff und Kohlenmonoxid eingespeist. Die Temperatur im Autoklaven wurde hierbei auf 115 °C gehalten. Nach 300 min wurde kein Gas mehr aufgenommen, und die Einspeisung beendet. Die NMR-Analyse erbrachte, daß 85 % der Undecyl-10-ensäure umgesetztwaren. Das Umsetzungsgemisch, das 11-Formylundecansäure enthielt, wurde nach Abzug des Lösungsmittels aus Aceton umkristallisiert. Die Ausbeute an 11-Formylundecansäure betrug 28,2 g = 37,3 %.

Beispiel 2

10 g 11-Formylundecansäure, wie in Beispiel 1 synthetisiert, gelöst in 50 ml Toluol, wurden bei 60 °C mit 30 mg Co(II)-acetat und 1 ml 25 %-iger Perpropionsäure versetzt und anschließend über 5 Stunden mit Sauerstoff begast. Beim Abkühlen fällt ein Niederschlag von 1,12-Dodecandisäure aus. Es konnten 9,7 g (90 %) isoliert werden.

Beispiel 3

Es wurde wie in Beispiel 1 verfahren, jedoch wurde das Reaktionsgemisch nach Beendigung der Gasaufnahme mit 0,7 g Co(II)-acetat und 100 ml Toluol versetzt auf 60 °C erwärmt und mit 2ml 25 %-iger Perpropionsäure versetzt. Anschließend wurde über 5 h mit Sauerstoff begast. Beim Abkühlen kristallisiert aus dem Reaktionsgemisch 1,12-Dodecandisäure aus. Die Ausbeute, bezogen auf eingesetzte Undecyl-10-ensäure, belief sich auf 70 %.

**Ansprüche**

1. Verfahren zur Herstellung von 1,12-Dodecandisäure durch Hydroformylierung einer ungesättigten Carboxylverbindung in Gegenwart eines Rhodiumkatalysators und Oxidation des dabei gebildeten Aldehyds mit Sauerstoff, dadurch gekennzeichnet, daß man Undecyl-10-en-säure in Gegenwart von Hydridotristriphenylphosphin-rhodiumcarbonyl kombiniert mit Triphenylphosphin und/oder Triphenylphosphit hydroformyliert, und die dabei gebildete 11-Formylundecansäure in Gegenwart von anorganischen oder organischen Co-(II)-salzen mit Sauerstoff oxidiert.

2. Verfahren gemäß Anspruch 1, dadurch gekennzeichnet, daß man die 11-Formylundecansäure aus dem Reaktionsgemisch abtrennt und anschließend oxidiert.

3. Verfahren gemäß Anspruch 1, dadurch gekennzeichnet, daß man die 11-Formylundecansäure im Reaktionsgemisch oxidiert.

4. Verfahren nach Anspruch 1, dadurch gekennzeichnet, daß man je Gewichtsteil der Undecyl-10-en-säure 0,002 bis 0,01 Gewichtsteile des Hydridotristriphenylphosphin-rhodium-carbonyls und 0,02 bis 0,3 Gewichtsteile des Triphenylphosphins oder Triphenylphosphits anwendet.

5. Verfahren nach den Ansprüchen 1 und 4, dadurch gekennzeichnet, daß man bei Temperaturen von 80 bis 140 °C und Drücken von 1 bis 12 bar hydroformyliert.

6. Verfahren nach den Ansprüchen 1 bis 3, dadurch gekennzeichnet, daß man bei Temperaturen von 10 bis 100 °C, vorzugsweise 40 bis 90 °C, oxidiert.

7. Verfahren nach den Ansprüchen 1 bis 3, 5 und 6, dadurch gekennzeichnet, daß man je Gewichtsteil der 11-Formylundecansäure 0,005 bis 0,09 Gewichtsteile des Co(II)-salzes und 0,002 bis 0,2 Gewichtsteile einer Percarbonsäure verwendet.

8. Verfahren nach den Ansprüchen 1 bis 3, 5, 6 und 7, dadurch gekennzeichnet, daß man Co(II)-acetat verwendet.